# EUROPEAN PATENT APPLICATION

(11) **EP 4 389 053 A1**
(43) Date of publication of application: **26.06.2024**
(21) Application number: 23217825.1
(22) Date of filing: 19.12.2023
(51) Int. Cl.: A61B 34/37, A61B 90/00, A61M 25/09

(54) **TORQUE LMITING ACTUATOR FOR ELONGATED MEDICAL DEVICE TORQUER**

(30) Priority: 21.12.2022 US 202263476397 P; 04.08.2023 US 202318365260
(71) Applicant: Corindus, Inc., Newton, MA 02466 (US)
(72) Inventor: ZIRPS, Christopher, Sharon, MA, 02067 (US); FALB, Peter, Hingham, MA, 02043 (US); HUANG, Zilong, Malden, MA, 02148 (US)
(74) Representative: Horn Kleimann Waitzhofer Schmid-Dreyer Patent- und Rechtsanwälte PartG mbB

(57) **Abstract**

A torquer releasably securing an elongated medical device thereto and including a torque limiting actuator limiting a torque applied to the torquer.

## Description

### CROSS-REFERENCE TO RELATED PATENT APPLICATIONS

This application claims priority to U.S. Provisional Application No. 63/476,397, filed December 21, 2022, the disclosure of which is incorporated herein by reference for all purposes.

### FIELD

The present invention relates generally to the field of robotic medical procedure systems and, in particular, to an elongated medical device torquer.

### BACKGROUND

Catheters and other elongated medical devices (EMDs) may be used for minimally invasive medical procedures for the diagnosis and treatment of diseases of various vascular systems, including neurovascular intervention (NVI) also known as neurointerventional surgery, percutaneous coronary intervention (PCI) and peripheral vascular intervention (PVI). These procedures typically involve navigating a guidewire through the vasculature, and via the guidewire advancing a catheter to deliver therapy. The catheterization procedure starts by gaining access into the appropriate vessel, such as an artery or vein, with an introducer sheath using standard percutaneous techniques. Through the introducer sheath, a sheath or guide catheter is then advanced over a diagnostic guidewire to a primary location such as an internal carotid artery for NVI, a coronary ostium for PCI, or a superficial femoral artery for PVI. A guidewire suitable for the vasculature is then navigated through the sheath or guide catheter to a target location in the vasculature. In certain situations, such as in tortuous anatomy, a support catheter or microcatheter is inserted over the guidewire to assist in navigating the guidewire. The physician or operator may use an imaging system (e.g., fluoroscope) to obtain a cine with a contrast injection and select a fixed frame for use as a roadmap to navigate the guidewire or catheter to the target location, for example, a lesion. Contrast-enhanced images are also obtained while the physician delivers the guidewire or catheter so that the physician can verify that the device is moving along the correct path to the target location. While observing the anatomy using fluoroscopy, the physician manipulates the proximal end of the guidewire or catheter to direct the distal tip into the appropriate vessels toward the lesion or target anatomical location and avoid advancing into side branches.

Robotic catheter-based procedure systems have been developed that may be used to aid a physician in performing catheterization procedures such as, for example, NVI, PCI and PVI. Examples of NVI procedures include coil embolization of aneurysms, liquid embolization of arteriovenous malformations and mechanical thrombectomy of large vessel occlusions in the setting of acute ischemic stroke. In an NVI procedure, the physician uses a robotic system to gain target lesion access by controlling the manipulation of a neurovascular guidewire and microcatheter to deliver the therapy to restore normal blood flow. Target access is enabled by the sheath or guide catheter but may also require an intermediate catheter for more distal territory or to provide adequate support for the microcatheter and guidewire. The distal tip of a guidewire is navigated into, or past, the lesion depending on the type of lesion and treatment. For treating aneurysms, the microcatheter is advanced into the lesion and the guidewire is removed and several embolization coils are deployed into the aneurysm through the microcatheter and used to block blood flow into the aneurysm. For treating arteriovenous malformations, a liquid embolic is injected into the malformation via a microcatheter. Mechanical thrombectomy to treat vessel occlusions can be achieved either through aspiration and/or use of a stent retriever. Depending on the location of the clot, aspiration is either done through an aspiration catheter, or through a microcatheter for smaller arteries. Once the aspiration catheter is at the lesion, negative pressure is applied to remove the clot through the catheter. Alternatively, the clot can be removed by deploying a stent retriever through the microcatheter. Once the clot has integrated into the stent retriever, the clot is retrieved by retracting the stent retriever and microcatheter (or intermediate catheter) into the guide catheter.

In PCI, the physician uses a robotic system to gain lesion access by manipulating a coronary guidewire to deliver the therapy and restore normal blood flow. The access is enabled by seating a guide catheter in a coronary ostium. The distal tip of the guidewire is navigated past the lesion and, for complex anatomies, a microcatheter may be used to provide adequate support for the guidewire. The blood flow is restored by delivering and deploying a stent or balloon at the lesion. The lesion may need preparation prior to stenting, by either delivering a balloon for pre-dilation of the lesion, or by performing atherectomy using, for example, a laser or rotational atherectomy catheter and a balloon over the guidewire. Diagnostic imaging and physiological measurements may be performed to determine appropriate therapy by using imaging catheters or fractional flow reserve (FFR) measurements.

In PVI, the physician uses a robotic system to deliver the therapy and restore blood flow with techniques similar to NVI. The distal tip of the guidewire is navigated past the lesion and a microcatheter may be used to provide adequate support for the guidewire for complex anatomies. The blood flow is restored by delivering and deploying a stent or balloon to the lesion. As with PCI, lesion preparation and diagnostic imaging may be used as well.

When support at the distal end of a catheter or guidewire is needed, for example, to navigate tortuous or calcified vasculature, to reach distal anatomical locations, or to cross hard lesions, an over-the-wire (OTW) catheter or coaxial system is used. An OTW catheter has a lumen for the guidewire that extends the full length of the catheter. This provides a relatively stable system because the guidewire is supported along the whole length. This system, however, has some disadvantages, including higher friction, and longer overall length compared to rapid-exchange catheters (see below). Typically to remove or exchange an OTW catheter while maintaining the position of the indwelling guidewire, the exposed length (outside of the patient) of guidewire must be longer than the OTW catheter. A 300 cm long guidewire is typically sufficient for this purpose and is often referred to as an exchange length guidewire. Due to the length of the guidewire, two operators are needed to remove or exchange an OTW catheter. This becomes even more challenging if a triple coaxial, known in the art as a tri-axial system, is used (quadruple coaxial catheters have also been known to be used). However, due to its stability, an OTW system is often used in NVI and PVI procedures. On the other hand, PCI procedures often use rapid exchange (or monorail) catheters. The guidewire lumen in a rapid exchange catheter runs only through a distal section of the catheter, called the monorail or rapid exchange (RX) section. With a RX system, the operator manipulates the interventional devices parallel to each other (as opposed to with an OTW system, in which the devices are manipulated in a serial configuration), and the exposed length of guidewire only needs to be slightly longer than the RX section of the catheter. A rapid exchange length guidewire is typically 180-200 cm long. Given the shorter length guidewire and monorail, RX catheters can be exchanged by a single operator. However, RX catheters are often inadequate when more distal support is needed.

### SUMMARY

In one embodiment a device for manipulating an elongated medical device releasably secures an elongated medical device (EMD) thereto and includes a torque limiting actuator limiting a torque applied to the torquer.

In one implementation the torque limiting actuator limits the torque applied to the torquer to a predetermined torque.

In one implementation the torque limiting actuator includes a manual operator and a clutch that prevents an application of torque to the torquer beyond a predetermined torque.

In one implementation the torque limiting actuator includes a knob being manually rotated about a longitudinal axis of the torquer, a biasing member between a portion of the knob and a first clutch member, wherein the first clutch member imparts torque to a second clutch member until a predetermined torque between the first clutch member and second clutch member is reached.

In one implementation the torque limiting actuator limits the torque applied to the actuator in a first direction but does not limit the torque applied to the actuator in a second opposite direction.

In one implementation the torquer includes a torquer body and a pusher movable within the torquer body to move at least a first pad in a direction perpendicular to a longitudinal axis of a torquer to pinch the EMD.

In one implementation a pinch force perpendicular to a longitudinal axis of the torquer between the torquer and the EMD is a function of the torque applied by the torque limiting actuator to the torquer.

In one implementation the clutch is a spring biased first gear that engages a second gear.

In one implementation the first gear slips relative to the second gear once a predetermined torque between the first gear and the second gear is exceeded.

In one implementation the torquer includes a second pad spaced from and movable toward and away from the first pad to releasably pinch the EMD.

In one implementation a biasing member includes a pair of arms spaced from one another and spaced from a longitudinal axis of the torquer.

In one implementation the torque limiting actuator includes a shaft having a portion threadedly secured to a body of the torquer and a distal portion operatively moving a pad into engagement with the EMD upon rotation of the shaft relative to the body.

In one implementation the torque limiting actuator includes a drive gear and a biasing member biasing the drive gear into engagement with a driven gear secured to the shaft, the drive gear.

In one implementation the torque limiting actuator includes a knob manually rotated relative to the body being releasably connected to the shaft upon an application of a predetermined torque.

In one implementation the torque limiting actuator provides an indication to a user, such as through haptic feedback in the form of an audible clicking sound and/or a vibration in a knob, once adequate torque has been applied to the torquer.

In one embodiment a torquer for an elongated medical device includes a body having a cavity defining a pathway; a first pad movable within the cavity; a biasing member separate from the first pad biasing the first pad relative to the body; an actuator movable relative to the body moving the first pad pinching and/or unpinching the elongated medical device with the first pad within the pathway; and a knob releasably connected to the actuator upon an application of a predetermined torque exceeding a predetermined value.

In one embodiment an EMD drive includes a robotic drive having a robotic drive longitudinal axis; a device module movable along the robotic drive longitudinal axis; a drive train coupling a motor to a driven member configured to rotate a torquer pinching an elongated medical device (EMD) about an EMD longitudinal axis, the torquer including a torque limiting actuator being manually accessible to a user when the torquer is in an in-use position in the drive module.

In one implementation the torquer may be moved from a first device module to a second device module.

In one implementation the torquer includes a torque limiting actuator that limits the torque applied to the drive train beyond a predetermined torque.

### BRIEF DESCRIPTION OF THE DRAWINGS

FIG 1 is a schematic view of an exemplary catheter procedure system.
FIG 2 is a schematic block diagram of an exemplary catheter procedure system.
FIG 3 is an exploded view of a cassette assembly and robotic drive and drive modules of a catheter procedure system.
FIG 4 is an isometric view of a torquer actuator.
FIG 5 is an exploded view of the torquer actuator of FIG 4.

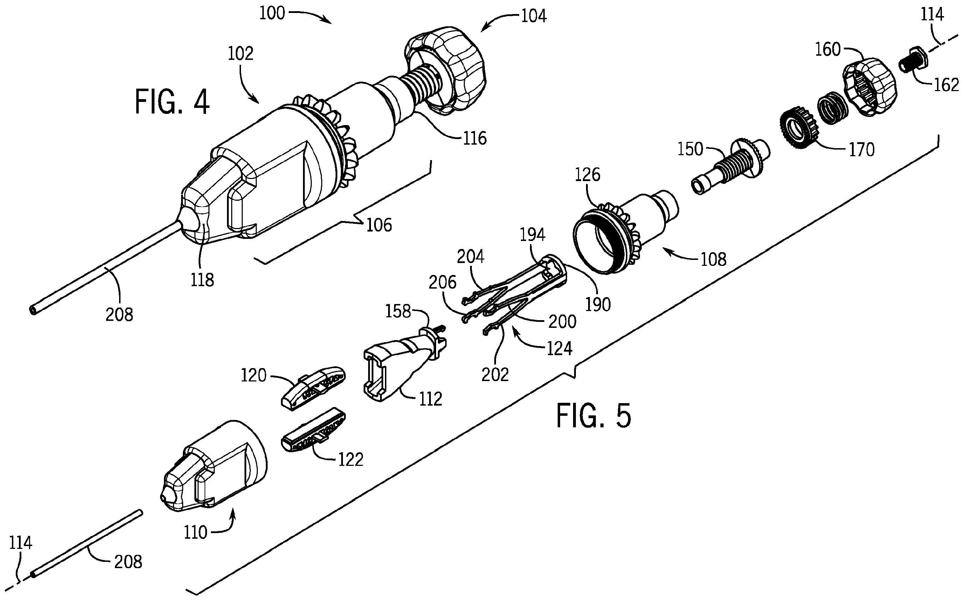

FIG 5 is a front plan view of the robotic drive and distal most drive module.

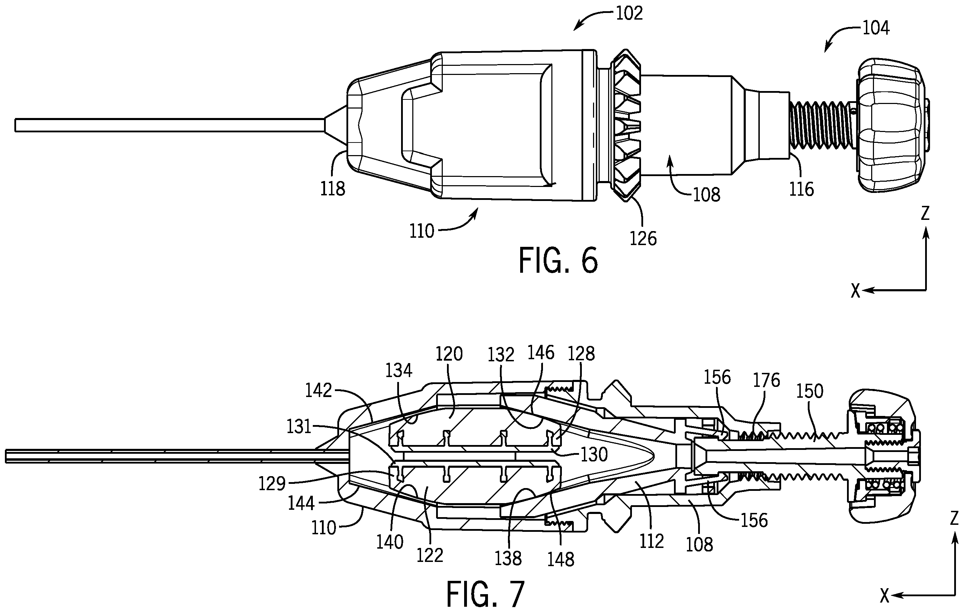

FIG 6 is a side plan view of the torquer actuator of FIG 4.
FIG 7 is a cross sectional view of the torquer actuator of FIG 6.

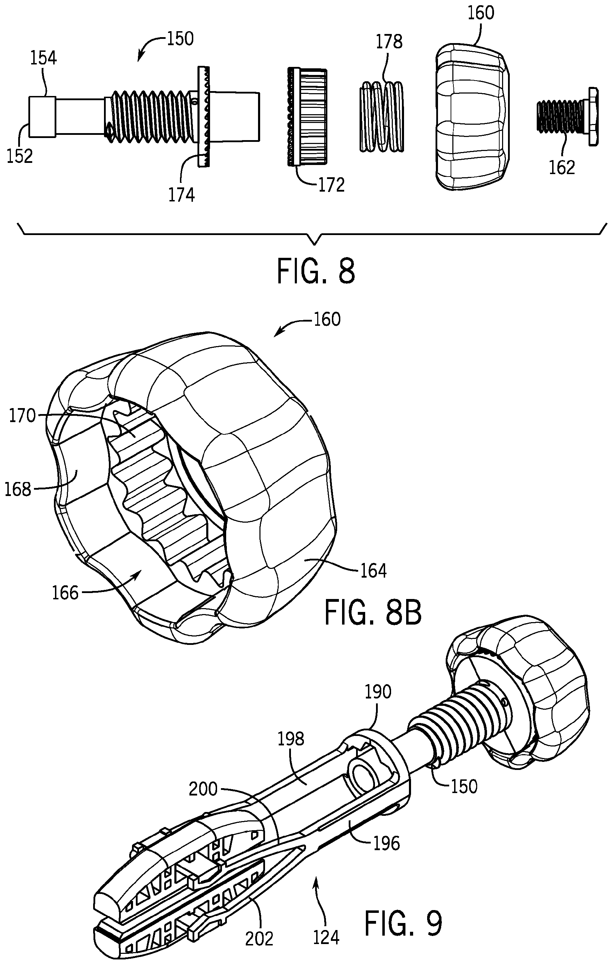

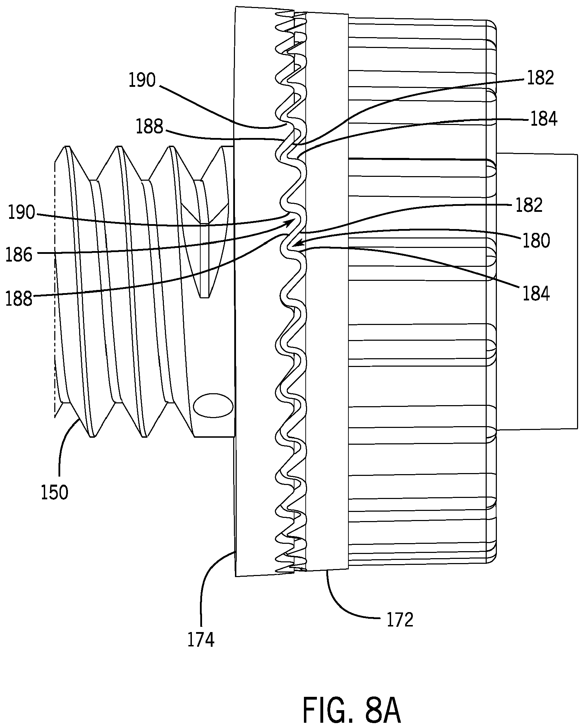

FIG 8 is an exploded view of a torque limiting knob assembly of the torquer actuator of FIG 4.
FIG 8A is a close-up for a portion of the torque limiting assembly.
FIG 8B is an isometric view of the knob of the torque limiting assembly.
FIG 9 is an isometric view of the pads and biasing member of the torquer actuator of FIG 4.

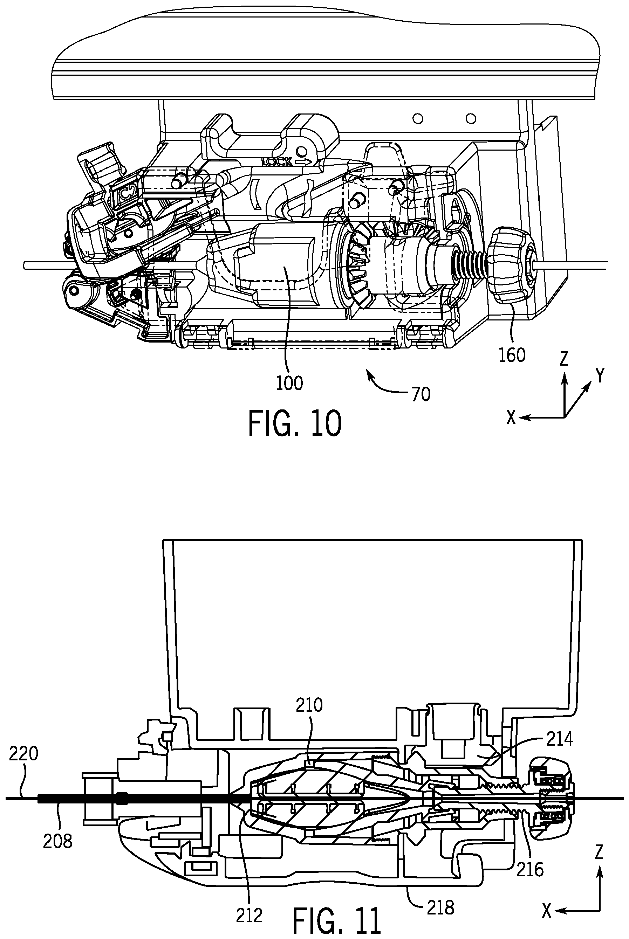

FIG 10 is an isometric view of the torquer actuator in a cassette of the catheter procedure system.
FIG 11 is a cross-sectional view of a torquer actuator illustrating various stop features.

### DETAILED DESCRIPTION OF THE EXAMPLE EMBODIMENTS

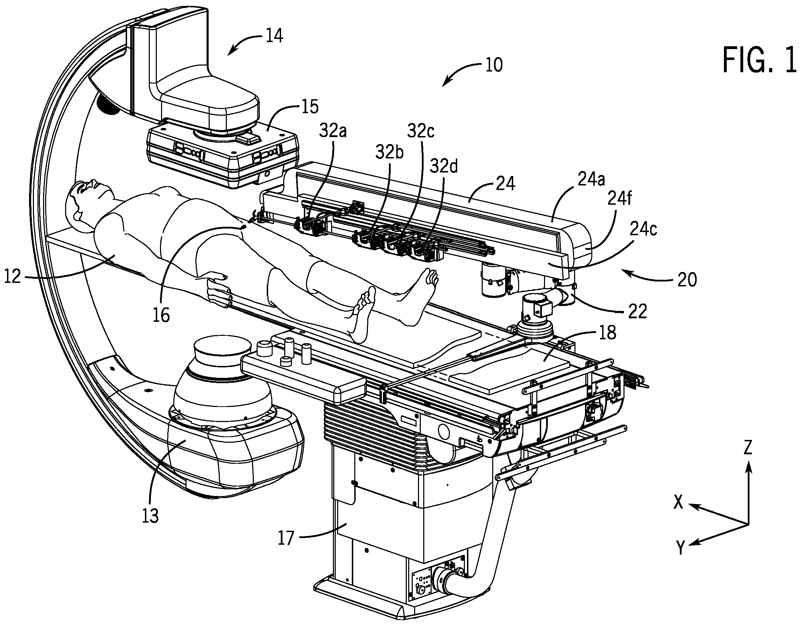

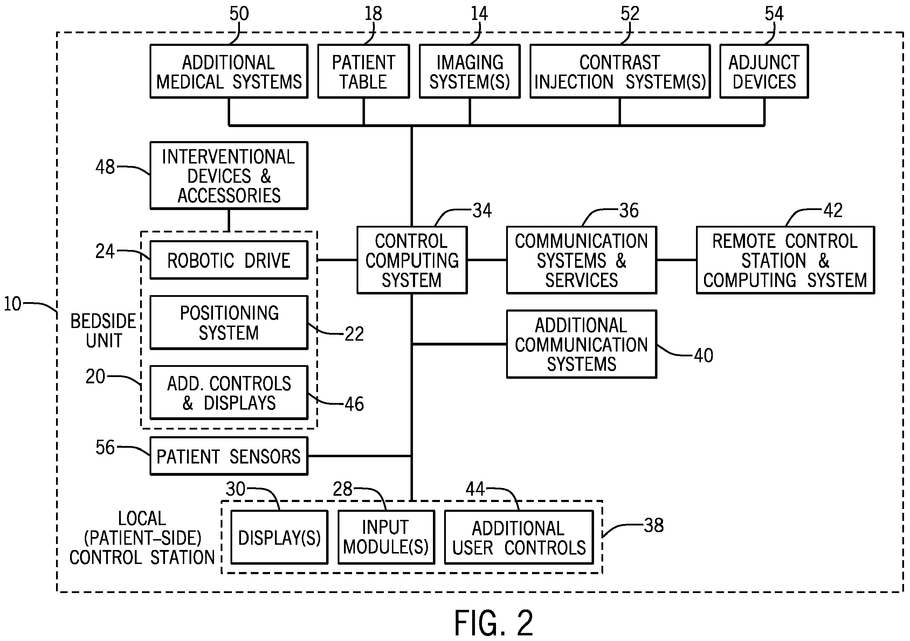

FIG. 1 is a perspective view of an example catheter-based procedure system 10 in accordance with an embodiment. Catheter-based procedure system 10 may be used to perform catheter-based medical procedures, e.g., percutaneous intervention procedures such as a percutaneous coronary intervention (PCI) (e.g., to treat STEMI), a neurovascular interventional procedure (NVI) (e.g., to treat an emergent large vessel occlusion (ELVO)), peripheral vascular intervention procedures (PVI) (e.g., for critical limb ischemia (CLI), etc.). Catheter-based medical procedures may include diagnostic catheterization procedures during which one or more catheters or other elongated medical devices (EMDs) are used to aid in the diagnosis of a patient's disease. For example, during one embodiment of a catheter-based diagnostic procedure, a contrast media is injected onto one or more arteries through a catheter and an image of the patient's vasculature is taken. Catheter-based medical procedures may also include catheter-based therapeutic procedures (e.g., angioplasty, stent placement, treatment of peripheral vascular disease, clot removal, arterial venous malformation therapy, treatment of aneurysm, etc.) during which a catheter (or other EMD) is used to treat a disease. Therapeutic procedures may be enhanced by the inclusion of adjunct devices 54 (shown in FIG. 2) such as, for example, intravascular ultrasound (IVUS), optical coherence tomography (OCT), fractional flow reserve (FFR), etc. It should be noted, however, that one skilled in the art would recognize that certain specific percutaneous intervention devices or components (e.g., type of guidewire, type of catheter, etc.) may be selected based on the type of procedure that is to be performed. Catheter-based procedure system 10 can perform any number of catheter-based medical procedures with minor adjustments to accommodate the specific percutaneous intervention devices to be used in the procedure.

Catheter-based procedure system 10 includes, among other elements, a bedside unit 20 and a control station (not shown). Bedside unit 20 includes a robotic drive 24 and a positioning system 22 that are located adjacent to a patient 12. Patient 12 is supported on a patient table 18. The positioning system 22 is used to position and support the robotic drive 24. The positioning system 22 may be, for example, a robotic arm, an articulated arm, a holder, etc. The positioning system 22 may be attached at one end to, for example, the patient table 18 (as shown in FIG. 1), a base, or a cart. The other end of the positioning system 22 is attached to the robotic drive 24. The positioning system 22 may be moved out of the way (along with the robotic drive 24) to allow for the patient 12 to be placed on the patient table 18. Once the patient 12 is positioned on the patient table 18, the positioning system 22 may be used to situate or position the robotic drive 24 relative to the patient 12 for the procedure. The position of the robotic drive in a position for the procedure is referred to herein as the robotic drive in-use position. In an embodiment, patient table 18 is operably supported by a pedestal 17, which is secured to the floor and/or earth. Patient table 18 is able to move with multiple degrees of freedom, for example, roll, pitch, and yaw, relative to the pedestal 17. Bedside unit 20 may also include controls and displays 46 (shown in FIG. 2). For example, controls and displays may be located on a housing of the robotic drive 24.

Generally, the robotic drive 24 may be equipped with the appropriate percutaneous interventional devices and accessories 48 (shown in FIG. 2) (e.g., guidewires, various types of catheters including but not limited to balloon catheters, stent delivery systems, stent retrievers, embolization coils, liquid embolics, aspiration pumps, device to deliver contrast media, medicine, hemostasis valve adapters, syringes, stopcocks, inflation device, etc.) to allow a user or operator to perform a catheter-based medical procedure via a robotic system by operating various controls such as the controls and inputs located at the control station. Bedside unit 20, and in particular robotic drive 24, may include any number and/or combination of components to provide bedside unit 20 with the functionality described herein. The robotic drive 24 includes a plurality of device modules 32a-d mounted to a rail or linear member. Each of the device modules 32a-d may be used to drive an EMD such as a catheter or guidewire. For example, the robotic drive 24 may be used to automatically feed a guidewire into a diagnostic catheter and into a guide catheter in an artery of the patient 12. One or more devices, such as an EMD, enter the body (e.g., a vessel) of the patient 12 at an insertion point 16 via, for example, an introducer sheath. Each device module 32a-d include a drive module and cassette removably attached to the drive module. Each drive module is movable along the robotic drive longitudinal axis with a bracket or stage. While FIG 1 illustrates four device modules it is contemplated that the number of device modules may be one or more.

Bedside unit 20 is in communication with the control station (not shown), allowing signals generated by the user inputs of the control station to be transmitted wirelessly or via hardwire to the bedside unit 20 to control various functions of bedside unit 20. As discussed below, control station 26 may include a control computing system 34 (shown in FIG. 2) or be coupled to the bedside unit 20 through the control computing system 34. Bedside unit 20 may also provide feedback signals (e.g., loads, speeds, operating conditions, warning signals, error codes, etc.) to the control station, control computing system 34 (shown in FIG. 2), or both. Communication between the control computing system 34 and various components of the catheter-based procedure system 10 may be provided via a communication link that may be a wireless connection, cable connections, or any other means capable of allowing communication to occur between components. The control station or other similar control system may be located either at a local site (e.g., local control station 38 shown in FIG. 2) or at a remote site (e.g., remote control station and computer system 42 shown in FIG. 2). Catheter procedure system 10 may be operated by a control station at the local site, a control station at a remote site, or both the local control station and the remote control station at the same time. At a local site, a user or operator and the control station are located in the same room or an adjacent room to the patient 12 and bedside unit 20. As used herein, a local site is the location of the bedside unit 20 and a patient 12 or subject (e.g., animal or cadaver) and the remote site is the location of a user or operator and a control station used to control the bedside unit 20 remotely. A control station (and a control computing system) at a remote site and the bedside unit 20 and/or a control computing system at a local site may be in communication using communication systems and services 36 (shown in FIG. 2), for example, through the Internet. In an embodiment, the remote site and the local (patient) site are away from one another, for example, in different rooms in the same building, different buildings in the same city, different cities, or other different locations where the remote site does not have physical access to the bedside unit 20 and/or patient 12 at the local site.

The control station generally includes one or more input modules 28 configured to receive user inputs to operate various components or systems of catheter-based procedure system 10. In the embodiment shown, control station allows the user or operator to control bedside unit 20 to perform a catheter-based medical procedure. For example, input modules 28 may be configured to cause bedside unit 20 to perform various tasks using percutaneous intervention devices (e.g., EMDs) interfaced with the robotic drive 24 (e.g., to advance, retract, or rotate a guidewire, advance, retract or rotate a catheter, inflate or deflate a balloon located on a catheter, position and/or deploy a stent, position and/or deploy a stent retriever, position and/or deploy a coil, inject contrast media into a catheter, inject liquid embolics into a catheter, inject medicine or saline into a catheter, aspirate on a catheter, or to perform any other function that may be performed as part of a catheter-based medical procedure). Robotic drive 24 includes various drive mechanisms to cause movement (e.g., axial and rotational movement) of the components of the bedside unit 20 including the percutaneous intervention devices.

In one embodiment, input modules 28 may include one or more touch screens, joysticks, scroll wheels, and/or buttons. In addition to input modules 28, the control station 26 may use additional user controls 44 (shown in FIG. 2) such as foot switches and microphones for voice commands, etc. Input modules 28 may be configured to advance, retract, or rotate various components and percutaneous intervention devices such as, for example, a guidewire, and one or more catheters or microcatheters. Buttons may include, for example, an emergency stop button, a multiplier button, device selection buttons and automated move buttons. When an emergency stop button is pushed, the power (e.g., electrical power) is shut off or removed to bedside unit 20. When in a speed control mode, a multiplier button acts to increase or decrease the speed at which the associated component is moved in response to a manipulation of input modules 28. When in a position control mode, a multiplier button changes the mapping between input distance and the output commanded distance. Device selection buttons allow the user or operator to select which of the percutaneous intervention devices loaded into the robotic drive 24 are controlled by input modules 28. Automated move buttons are used to enable algorithmic movements that the catheter-based procedure system 10 may perform on a percutaneous intervention device without direct command from the user or operator. In one embodiment, input modules 28 may include one or more controls or icons (not shown) displayed on a touch screen (that may or may not be part of a display), that, when activated, causes operation of a component of the catheter-based procedure system 10. Input modules 28 may also include a balloon or stent control that is configured to inflate or deflate a balloon and/or deploy a stent. Each of the input modules 28 may include one or more buttons, scroll wheels, joysticks, touch screen, etc. that may be used to control the particular component or components to which the control is dedicated. In addition, one or more touch screens may display one or more icons (not shown) related to various portions of input modules 28 or to various components of catheter-based procedure system 10.

Catheter-based procedure system 10 also includes an imaging system 14. Imaging system 14 may be any medical imaging system that may be used in conjunction with a catheter based medical procedure (e.g., non-digital X-ray, digital X-ray, CT, MRI, ultrasound, etc.). In an exemplary embodiment, imaging system 14 is a digital X-ray imaging device that is in communication with the control station. In one embodiment, imaging system 14 may include a C-arm (shown in FIG. 1) that allows imaging system 14 to partially or completely rotate around patient 12 in order to obtain images at different angular positions relative to patient 12 (e.g., sagittal views, caudal views, anterior-posterior views, etc.). In one embodiment imaging system 14 is a fluoroscopy system including a C-arm having an X-ray source 13 and a detector 15, also known as an image intensifier.

Imaging system 14 may be configured to take X-ray images of the appropriate area of patient 12 during a procedure. For example, imaging system 14 may be configured to take one or more X-ray images of the head to diagnose a neurovascular condition. Imaging system 14 may also be configured to take one or more X-ray images (e.g., real time images) during a catheter-based medical procedure to assist the user or operator of control station 26 to properly position a guidewire, guide catheter, microcatheter, stent retriever, coil, stent, balloon, etc. during the procedure. The image or images may be displayed on display 30. For example, images may be displayed on a display to allow the user or operator to accurately move a guide catheter or guidewire into the proper position.

In order to clarify directions, a rectangular coordinate system is introduced with X, Y, and Z axes. The positive X axis is oriented in a longitudinal (axial) distal direction, that is, in the direction from the proximal end to the distal end, stated another way from the proximal to distal direction. The Y and Z axes are in a transverse plane to the X axis, with the positive Z axis oriented up, that is, in the direction opposite of gravity, and the Y axis is automatically determined by right-hand rule. As used herein the X axis extends along a longitudinal axis of the robotic drive 24. Since in an in-use position the robotic housing may be at an angle with respect to the horizontal plane perpendicular to the direction of gravity the X, Y and Z axes are defined by robotic drive 24. Referring to FIG 1, robotic drive 24 includes a housing having a top or first member 24a parallel to the X-Y plane; a bottom or second member parallel to and spaced from the first member 24a; a front or third member 24c substantially perpendicular and extending between first member 24a and the second member, the third member facing a user when robotic drive 24 is in the in-use position or orientation illustrated in FIG 1. A fourth member is spaced from and substantially parallel to third member 24c and perpendicular to first member 24a and the second member 24b. It is contemplated that other shapes of the robotic drive housing may be used. In which case first member 24a would be the upper member, the second member would be the lower or bottom member, front or third member 24c would be the portion facing a user in an in-use position during a surgical procedure, and the fourth member is the portion facing away from the user in the in-use position during a surgical procedure. Robotic drive 24 further includes a distal region 24e and a proximal region 24f. Where the distal region 24e is closer to the entry point of the patient through which the EMD will be introduced and the proximal region 24f is furthest from the entry point of the patient through which the EMD will be introduced.

FIG. 2 is a block diagram of catheter-based procedure system 10 in accordance with an example embodiment. Catheter-procedure system 10 may include a control computing system 34. Control computing system 34 may physically be, for example, part of a control station. Control computing system 34 may generally be an electronic control unit suitable to provide catheter-based procedure system 10 with the various functionalities described herein. For example, control computing system 34 may be an embedded system, a dedicated circuit, a general-purpose system programmed with the functionality described herein, etc. Control computing system 34 is in communication with bedside unit 20, communications systems and services 36 (e.g., Internet, firewalls, cloud services, session managers, a hospital network, etc.), a local control station 38, additional communications systems 40 (e.g., a telepresence system), a remote control station and computing system 42, and patient sensors 56 (e.g., electrocardiogram (ECG) devices, electroencephalogram (EEG) devices, blood pressure monitors, temperature monitors, heart rate monitors, respiratory monitors, etc.). The control computing system is also in communication with imaging system 14, patient table 18, additional medical systems 50, contrast injection systems 52 and adjunct devices 54 (e.g., IVUS, OCT, FFR, etc.). The bedside unit 20 includes a robotic drive 24, a positioning system 22 and may include additional controls and displays 46. As mentioned above, the additional controls and displays may be located on a housing of the robotic drive 24. Interventional devices and accessories 48 (e.g., guidewires, catheters, etc.) interface to the bedside unit 20. In an embodiment, interventional devices and accessories 48 may include specialized devices (e.g., IVUS catheter, OCT catheter, FFR wire, diagnostic catheter for contrast, etc.) which interface to their respective adjunct devices 54, namely, an IVUS system, an OCT system, and FFR system, etc.

In various embodiments, control computing system 34 is configured to generate control signals based on the user's interaction with input modules 28 (e.g., of a control station such as a local control station 38 or a remote control station 42) and/or based on information accessible to control computing system 34 such that a medical procedure may be performed using catheter-based procedure system 10. The local control station 38 includes one or more displays 30, one or more input modules 28, and additional user controls 44. The remote control station and computing system 42 may include similar components to the local control station 38. The remote 42 and local 38 control stations can be different and tailored based on their required functionalities. The additional user controls 44 may include, for example, one or more foot input controls. The foot input control may be configured to allow the user to select functions of the imaging system 14 such as turning on and off the X-ray and scrolling through different stored images. In another embodiment, a foot input device may be configured to allow the user to select which devices are mapped to scroll wheels included in input modules 28. Additional communication systems 40 (e.g., audio conference, video conference, telepresence, etc.) may be employed to help the operator interact with the patient, medical staff (e.g., angio-suite staff), and/or equipment in the vicinity of the bedside.

Catheter-based procedure system 10 may be connected or configured to include any other systems and/or devices not explicitly shown. For example, catheter-based procedure system 10 may include image processing engines, data storage and archive systems, automatic balloon and/or stent inflation systems, medicine injection systems, medicine tracking and/or logging systems, user logs, encryption systems, systems to restrict access or use of catheter-based procedure system 10, etc.

As mentioned, control computing system 34 is in communication with bedside unit 20 which includes a robotic drive 24, a positioning system 22 and may include additional controls and displays 46 and may provide control signals to the bedside unit 20 to control the operation of the motors and drive mechanisms used to drive the percutaneous intervention devices (e.g., guidewire, catheter, etc.). The various drive mechanisms may be provided as part of a robotic drive 24.

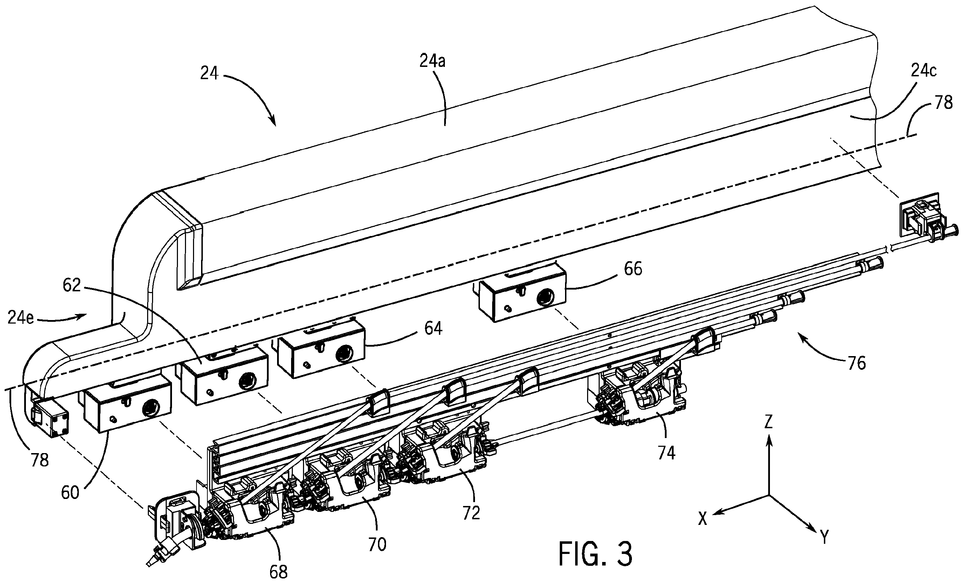

Referring to FIG 3 device module 32a includes a first drive module 60 and a first cassette 68. Device module 32b includes a second drive module 62 and a second cassette 70. Device module 32c includes a third drive module 64 and a third cassette 72. Device module 32d includes a fourth drive module 66 and a fourth cassette 74. In one implementation first cassette 68, second cassette 70, third cassette 72 and fourth cassette 74 are shipped together as a multi-unit cassette assembly. In one implementation the multi-unit cassette assembly 76 allows for each of the cassettes to be removably connected to their respective drive modules while slidably connected together. In one implementation each of the multiple device modules 32a-d may be independently actuated to move linearly along a linear member within robotic drive 24. Each device module 32a-d may independently move relative to each other and the linear member in the robotic drive. The drive mechanism moves each device module along a longitudinal axis 78 of robotic drive 24, also referred to herein as the robotic drive longitudinal axis 78. Robotic drive longitudinal axis 78 may extend along the linear member such as a screw drive along which device modules move or may be defined along another axis that is parallel to the linear member along which the device modules move. Referring to FIG 3 each cassette 68-74 are generally vertically oriented in the XZ plane. Each cassette 68-74 has a length along the X axis or parallel to longitudinal axis 78 that is greater than the width of each cassette along the Y axis or perpendicular to the Y axis. PCT International Publication No. WO 2021/011533, which is incorporated herein by reference in its entirety, discloses a cassette positioned in a generally horizontal position in the XY plane. The distinction between the vertical and horizontal of the cassettes is described in PCT International Publication No. WO 2021/011554 and incorporated herein by reference in its entirety.

Referring to WO 2021/011554, in one implementation, the drive mechanism includes independent stage translation motors coupled to each device module and a stage drive mechanism such as a lead screw via a rotating nut, a rack via a pinion, a belt via a pinion or pulley, a chain via a sprocket, or the stage translation motors 64a-d may be linear motors themselves. The drive mechanism provides for advancement and retraction of the device modules. Examples of such drive mechanisms are described in WO 2021/011533.

To prevent contaminating the patient with pathogens, healthcare staff use aseptic techniques in a room housing the bedside unit 20 and the patient 12 or subject (shown in FIG. 1). A room housing the bedside unit 20 and patient 12 may be, for example, a cath lab or an angio suite. Aseptic technique consists of using sterile barriers, sterile equipment, proper patient preparation, environmental controls and contact guidelines. Accordingly, all EMDs and interventional accessories are sterilized and can only be in contact with either sterile barriers or sterile equipment. In an embodiment, a sterile drape (not shown) is placed over the non-sterile robotic drive 24. Each cassette 68-74 is sterilized and acts as a sterile interface between the draped robotic drive 24 and at least one EMD. Each cassette 68-74 can be designed to be sterile for single use or to be re-sterilized in whole or part so that the cassette 68-74 or its components can be used in multiple procedures.

Distal and Proximal: The terms distal and proximal define relative locations of two different features. With respect to a robotic drive the terms distal and proximal are defined by the position of the robotic drive in its intended use relative to a patient. When used to define a relative position, the distal feature is the feature of the robotic drive that is closer to the patient than a proximal feature when the robotic drive is in its intended in-use position. Within a patient, any vasculature landmark further away along the path from the access point is considered more distal than a landmark closer to the access point, where the access point is the point at which the EMD enters the patient. Similarly, the proximal feature is the feature that is farther from the patient than the distal feature when the robotic drive in its intended in-use position. When used to define direction, the distal direction refers to a path on which something is moving or is aimed to move or along which something is pointing or facing from a proximal feature toward a distal feature and/or patient when the robotic drive is in its intended in-use position. The proximal direction is the opposite direction of the distal direction. By way of examples referring to FIG 1, a robotic device is shown from the viewpoint of an operator facing a patient. In this arrangement, the distal direction is along the positive X coordinate axis and the proximal direction is along the negative X coordinate axis.

Longitudinal axis: The term longitudinal axis of a member (for example, an EMD or other element in the catheter-based procedure system) is the line or axis along the length of the member that passes through the center of the transverse cross section of the member in the direction from a proximal portion of the member to a distal portion of the member. For example, the longitudinal axis of a guidewire is the central axis in the direction from a proximal portion of the guidewire toward a distal portion of the guidewire even though the guidewire may be non-linear in the relevant portion.

Axial Movement: The term axial movement of a member refers to translation of the member along the longitudinal axis of the member. When the distal end of an EMD is axially moved in a distal direction along its longitudinal axis into or further into the patient, the EMD is being advanced. When the distal end of an EMD is axially moved in a proximal direction along its longitudinal axis out of or further out of the patient, the EMD is being withdrawn.

Rotational Movement: The term rotational movement of a member refers to the change in angular orientation of the member about the local longitudinal axis of the member. Rotational movement of an EMD corresponds to clockwise or counterclockwise rotation of the EMD about its longitudinal axis due to an applied torque.

Axial and Lateral Insertion: The term axial insertion refers to inserting a first member into a second member along the longitudinal axis of the second member. An EMD that is axially loaded in a collet is axially inserted in the collet. An example of axial insertion could be referred to as back loading a catheter on the proximal end of a guidewire. The term lateral insertion refers to inserting a first member into a second member along a direction in a plane perpendicular to the longitudinal axis of the second member. This can also be referred to as radial loading or side loading. Stated another way, lateral insertion refers to inserting a first member into a second member along a direction that is parallel to the radius and perpendicular to the longitudinal axis of the second member.

Up/Down; Front/Rear; Inwardly/Outwardly: The terms top, up, and upper refer to the general direction away from the direction of gravity and the terms bottom, down, and lower refer to the general direction in the direction of gravity. The term front refers to the side of the robotic drive that faces a bedside user and away from the positioning system, such as the articulating arm. The term rear refers to the side of the robotic drive that is closest to the positioning system, such as the articulating arm. The term inwardly refers to the inner portion of a feature. The term outwardly refers to the outer portion of a feature.

Stage: The term stage refers to a member, feature, or device that is used to couple a device module to the robotic drive. For example, the stage may be used to couple the device module to a rail or linear member of the robotic drive.

Drive Module: The term drive module generally refers to the part (e.g., the capital part) of the robotic drive system that normally contains one or more motors with drive couplers that interface with the cassette.

Device Module: The term device module refers to the combination of a drive module and a cassette.

Cassette: The term cassette generally refers to the part (non-capital, consumable or sterilizable unit) of the robotic drive system that normally is the sterile interface between a drive module and at least one EMD (directly) or through a device adapter (indirectly).

Shaft (Distal) Driving: The term shaft (distal) driving refers to holding on to and manipulating an EMD along its shaft. In one example the on-device adapter is normally placed just proximal of the hub or Y-connector the device is inserted into. If the location of the on-device adapter is at the proximity of an insertion point (to the body or another catheter or valve), shaft driving does not typically require anti-buckling features. (It may include anti-buckling features to improve drive capability.)

Collet: The term collet refers to a device that can releasably fix a portion of an EMD. The term fixed here means no intentional relative movement of the collet and EMD during operation. In one embodiment the collet includes at least two members that move rotationally relative to each other to releasably fix the EMD to at least one of the two members. In one embodiment the collet includes at least two members that move axially (along a longitudinal axis) relative to each other to releasably fix the EMD to at least one of the two members. In one embodiment the collet includes at least two members that move rotationally and axially relative to each other to releasably fix the EMD to at least one of the two members.

Fixed: The term fixed means no intentional relative movement of a first member with respect to a second member during operation.

Pinch/Unpinch: The term pinch refers to releasably fixing an EMD to a member such that the EMD and member move together when the member moves. The term unpinch refers to releasing the EMD from a member such that the EMD is no longer fixed to a member but unfixed to that member and the EMD moves independently of the member.

On-Device Adapter: The term on-device adapter refers to a sterile apparatus capable of releasably pinching an EMD to provide a driving interface. The on-device adapter is also known as an end-effector or EMD capturing device. In one non-limiting embodiment the on-device adapter is a collet that is operatively controlled robotically to rotate the EMD about its longitudinal axis, to pinch and/or unpinch the EMD to the collet, and/or to translate the EMD along its longitudinal axis. In one embodiment the on-device adapter is a hub-drive mechanism such as a gear located on the hub of an EMD.

EMD: The term elongated medical device (EMD) refers to, but is not limited to, catheters (e.g., guide catheters, microcatheters, balloon/stent catheters), wire-based devices (e.g., guidewires, embolization coils, stent retrievers, etc.), and medical devices comprising any combination of these. In one example a wire-based EMD includes but is not limited to guidewires, microwires, a proximal pusher for embolization coils, stent retrievers, self-expanding stents, and flow divertors. Typically wire-based EMD's do not have a hub or handle at its proximal terminal end. In one embodiment the EMD is a catheter having a hub at a proximal end of the catheter and a flexible shaft extending from the hub toward the distal end of the catheter, wherein the shaft is more flexible than the hub. In one embodiment the catheter includes an intermediary portion that transitions between the hub and the shaft that has an intermediate flexibility that is less rigid than the hub and more rigid than the shaft. In one embodiment the intermediary portion is a strain relief.

Hub (Proximal) Driving: The term hub driving, or proximal driving refers to holding on to and manipulating an EMD from a proximal position (e.g., a geared adapter on a catheter hub). In one embodiment, hub driving refers to imparting a force or torque to the hub of a catheter to translate and/or rotate the catheter. Hub driving may cause the EMD to buckle and thus hub driving often requires anti-buckling features. For devices that do not have hubs or other interfaces (e.g., a guidewire), device adapters may be added to the device to act as an interface for the device module. In one embodiment, an EMD does not include any mechanism to manipulate features within the catheter such as wires that extend from the handle to the distal end of the catheter to deflect the distal end of the catheter.

Sterilizable Unit: The term sterilizable unit refers to an apparatus that is capable of being sterilized (free from pathogenic microorganisms). This includes, but is not limited to, a cassette, consumable unit, drape, device adapter, and sterilizable drive modules/units (which may include electromechanical components). Sterilizable Units may come into contact with the patient, other sterile devices, or anything else placed within the sterile field of a medical procedure.

Sterile Interface: The term sterile interface refers to an interface or boundary between a sterile and non-sterile unit. For example, a cassette may be a sterile interface between the robotic drive and at least one EMD.

Consumable: The term consumable refers to a sterilizable unit that normally has a single use in a medical procedure. The unit could be a reusable consumable through a re-sterilization process for use in another medical procedure.

Gear: The term gear may be a bevel gear, spiral bevel gear, spur gear, miter gear, worm gear, helical gear, rack and pinon, screw gear, internal gear such as a sun gear, involute spline shafts and bushing, or any other type of gears known in the art.

Referring to FIG 4 a torquer actuator 100 includes a torquer 102 and a torque limiting actuator 104. Torquer actuator 100 includes a housing 106 formed from a proximal housing member 108 that is operatively connected to a distal housing member 110. A pusher 112 is movably received within housing 106 along a torquer longitudinal axis 114 between a proximal end 116 of housing and distal end 118 of housing 106. A first pad 120 and a second pad 122 move toward and away from torquer longitudinal axis 114 to releasably pinch a shaft of an elongated medical device (EMD). A biasing member 124 biases first pad 120 and second pad 122 away from one another. Movement of pusher 112 from the proximal end 116 toward distal end 118 forces first pad 120 and second pad 122 toward one another and provides sufficient force to overcome the biasing force of biasing member 124. Movement of pusher 112 from distal end 118 toward proximal end 116 permits biasing member 124 to bias first pad 120 and second pad 122 away from one another and away from torquer longitudinal axis 114.

In one implementation first pad 120 includes a first portion 128 and a second portion 130 contacting the EMD in an engaged position. First portion 128 of first pad 120 includes an outer surface having a proximal ramp 132 and a distal ramp 134. Similarly, second pad 122 includes a first portion 129and a second portion 131 that contacts the EMD in an engaged position. First portion 129 includes a proximal ramp 138 and a distal ramp 140.

Distal housing member 110 includes a first ramp 142 and a second ramp 144. Pusher 112 includes a first ramp 146 and a second ramp 148. As pusher 112 is moved from a proximal position toward a distal position, first ramp 146 and second ramp 148 of pusher 112 contacts proximal ramp 132 and second ramp 148 of first pad 120 and second pad 122 respectively. Similarly, distal ramp 134 and distal ramp 140 contacts first ramp 142 and second ramp 144 of distal housing member 110 respectively. The contacts of the ramp portions force first pad 120 and second pad 122 toward one another in a generally perpendicular direction to torquer longitudinal axis 114 thereby pinching an EMD therebetween. The operation of a torquer for an elongated medical device is described in publication WO 2022/154977 entitled Torquer For An Elongated Medical Device and incorporated herein in its entirety.

Pusher 112 is moved distally within distal housing member 110 by manipulation of torque limiting actuator 104. Referring to FIG 7 and FIG 8 torque limiting actuator 104 includes a shaft 150 that is threadedly engaged with proximal housing member 108. Shaft 150 includes a distal end that is operatively connected to pusher 112 such that movement of shaft 150 in a distal direction moves pusher 112 in a distal direction and movement of shaft 150 in a proximal direction moves pusher 112 in a proximal direction. Pusher 112 has a distal end 152 that pushes a proximal end 158 pusher 112 in a distal direction. Pusher /includes a pair of arms 156 that engage a distal hub 154 of shaft 150 such that when shaft 150 is moved in a proximal direction, pusher 112 also moves in a proximal direction.

Torque limiting actuator 104 includes a knob 160 that is secured to shaft 150 with a fastener 162. Referring to FIG 8B, knob 160 includes an outer surface 164 that an operator manipulates and an interior cavity 166 that is defined by a cavity wall 168. In one implementation cavity wall includes a profile defined by a plurality of spaced ribs 170 that positively engage with a profile on a drive gear 172 that is positioned within cavity 166 and rotates with knob 160. Shaft 150 includes a driven gear 174 that is engaged with drive gear 172. Rotation of knob 160 in a first direction results in rotation of drive gear 172 which in turn rotates driven gear 174 and shaft 150 in a first direction. As knob 160 is rotated in the first direction shaft 150 moves in a distal direction within the threaded region 176 of proximal housing member 108. Movement in the distal direction then moves pusher 112 in a distal direction which causes first pad 120 and second pad 122 to move toward one another to pinch the EMD. In one implementation the profile of the cavity wall is a plurality of splines that engage with mating splines of a housing supporting drive gear 172. The mating splines prohibit rotational movement between knob 160 and drive gear 172 but allow for axial movement along torquer longitudinal axis 114 between knob 160 and drive gear 172.

A biasing member 178 is positioned within cavity 166 of knob 160 and acts to bias drive gear 172 into engagement with driven gear 174. Referring to FIG 8A driven gear 174 and drive gear 172 are face gears in that they face one another and rotate about a common axis, in this case, drive gear 172 and 174 rotate about torquer longitudinal axis 114. Drive gear 172 includes a plurality of gear teeth 180 having a first face 182 and a second face 184 where the angle of first face 182 is less than the angle of second face 184. In one implementation the angle of first face 182 is equal to or greater than the angle of second face 184. Similarly, driven gear 174 has a plurality of gear teeth 186 having a first face 188 and a second face 190 that engage with first face 182 and second face 184 of gear teeth 180.

Drive gear 172 and driven gear 174 act as a clutch, wherein drive gear 172 is a first clutch plate and driven gear 174 is a second clutch plate. When the torque exceeds a predetermined value first face 182 of gear teeth 180 rides up and over a corresponding first face 188 of gear teeth 186 resulting in drive gear 172 slipping with respect to driven gear 174. Stated another way the manner in which the gear teeth ride over one another rather than stay engaged during the application of a predetermined torque is referred to herein as slip or slipping.

Upon rotation of knob 160 in the first direction shaft 150 will continue to move first pad 120 and second pad 122 toward one another to pinch the EMD until the torque required to continue to move first pad 120 toward second pad 122 exceeds a predetermined force. Once the predetermined force is reached the spring force of biasing member 178 will no longer be sufficient to maintain gear teeth 180 to impart movement to gear teeth 186. The predetermined force rotation of knob 160 in the first direction will result in gear teeth 180 sliding over gear teeth 186, providing an audible clicking sound as well as tactile haptic feedback to the user, as the first face 182 of gear teeth 180 slide up and over first face 188 of gear teeth 186. In this manner torque limiting actuator 104 acts as a clutch that limits the amount of force and torque that can be applied to the EMD as first pad 120 and second pad 122 pinch the EMD even if the operator continues to turn knob 160 in the first direction once the predetermined force is reached.

As noted, the angle of second face 184 of gear teeth 180 and second face 190 of gear teeth 186 is greater than the angle of first face 182 and first face 188 of gear teeth 180 and gear teeth 186 respectively. In one implementation the angle of second face 184 and second face 190 is less than or equal to the angle of first face 182 and first face 188. The angle is sufficient to prevent second face 184 of gear teeth 180 sliding up and over the second face 190 of gear teeth 186 when knob 160 is rotated in a second direction opposite the first direction. In one implementation the rotation in the first direction is a clockwise (CW) direction as is the convention for tightening / engaging the torquer to the EMD and the second direction is a counterclockwise (CCW) direction which is the convention for loosening / disengaging the torquer from the EMD. Stated another way rotating in the counter-clockwise direction will cause the pads to open. Tightening and loosening of the torque device occurs in two primary scenarios: in free space with the torque device held in the operator's hands, and mounted in the disposable cassette, with the cover closed.

Housing 106 includes a gear 126 operatively secured to housing 106 that is driven by an actuator to rotate torquer actuator 100 along with an EMD 220 once EMD 220 has been secured to torquer 102.

Referring to FIG 9 biasing member 124 includes a base portion 190 having an aperture 194 that receives a portion of shaft 150. Shaft 150 and biasing member 124 are free to move along torquer longitudinal axis 114 independently of one another. Biasing member 124 includes a first arm 196 and a second arm 198 that are spaced from one another and spaced from torquer longitudinal axis 114. First arm 196 and second arm 198 extend along the outside of pusher 112. First arm 196 includes a first branch 200 and a second branch 202 that engage with a portion on a first side of first pad 120 and second pad 122 respectively. Similarly, second arm 198 includes a first branch 204 and a second branch 206 that engage with a portion on a second side of first pad 120 and second pad 122. Where the first side and second sides of first pad 120 and second pad 122 are spaced from and are on opposite directions of torquer longitudinal axis 114. Branches 200, 202, 204 and 206 are preloaded to bias first pad 120 and second pad 122 away from one another in both an engaged position in which first pad 120 and second pad 122 pinch an EMD and in the disengaged position which first pad 120 and second pad 122 are not engaged with and pinching an EMD. In one implementation there is only one arm 196 and not a second arm 198. In one implementation more than two arms are used. Branch 200 and branch 202 are biased to push apart pad 120 and pad 122 away from one another.

Referring to FIG 10 torquer actuator 100 is positioned within cassette 70 such that knob 160 extends outside of cassette 70 such that knob 160 can be manipulated by a user to rotate knob 160 in both the first CW direction and the second CCW direction. Note that torquer actuator 100 includes a guide tube 208 extending from the distal end of distal housing member 110 to guide the EMD through cassette 70.

Referring to FIG 11, stop members be used in conjunction with torque limiting actuator 104 or independently of torque limiting actuator 104 to limit the movement of pusher 112 in the distal direction and thereby limiting the amount of force that can be applied between first pad 120 and second pad 122. A pusher stop member 210 may be placed either on a distal portion of pusher 112 or on a portion of distal housing member 110 that would prohibit pusher 112 from moving in a distal direction once stop member 210 is contacted. First stop member 210 would limit the movement of pusher 112 beyond a certain point distally and therefore would limit the amount of force applied to EMD 220.

In one implementation a second pad stop member 212 may be placed either on an internal portion of distal housing member 110 that would contact one or both of a first pad 120 and second pad 122 to prohibit the pads from moving distally and therefore would limit the movement of the pads toward one another limiting the force applied to EMD 220.

In one implementation an actuator 214 that drives gear 126 may have a built in torque limiting mechanism that is either mechanical or electromechanical and controlled by a controller.

In one implementation a knob stop 216 may be placed on either knob 160 or on proximal housing member 108 that would limit the distance that shaft 150 could extend into housing 106 thereby limiting the distal travel of pusher 112, first pad 120 and second pad 122 thereby limiting the movement of first pad 120 and second pad 122 toward one another and as a result limiting the pinch force applied to the EMD by the pads.

In one implementation cover 218 is configured to move automatically from a closed in-use position to an open position upon a torque applied to torquer actuator 100 that exceeds a predetermined torque. This would provide a visual indication that the torque has exceeded a predetermined force. The opening of cover 218 along with the audible and tactile feedback provides visual feedback that the predetermined torque has been reached.

The devices described herein provide a number of features. First torque limiting actuator 104 provides to a user an audible and/or tactile feedback that they have applied adequate torque to the torquer to ensure the adequate torque and force can be applied to the inserted Elongated Percutaneous Device(EMD). Once the predetermined torque is reached the user will be alerted via the audible and tactile feedback of the first gear slipping over the second gear. This eliminates the case in which too little torque is applied resulting in poor performance by the EMD slipping within the torquer as the torquer is rotated.

Torque limiting actuator 104 limits the torque that can be applied to torquer 102. This allows lightweight and low-cost materials/processes, such as injection molded plastics to be used in the design. The torque limiting knob 160 prevents stronger users from damaging the torque device, while providing a large enough diameter that weaker users can provide the needed knob torque. Where second portion 130 is an elastomeric contact pad, limiting the torque applied to the torquer and therefore the force applied by the pads and the EMD avoids damaging coated EMDs used in the procedure. This type of design requires high forces and low contact friction between the sliding elements. By limiting the torque applied the coating of the EMDs can be protected and not damaged.

Torquer actuator 100 acts to protect components of robotic drive 24 from high torques. Torquer actuator 100 is tightened in place in the disposable cassette mounted on the system robot. High torque could damage the robotic drive, preventing use of the system. The torque limiting actuator 104 limits the torque applied to torquer actuator 100 and therefore also limits the torque applied to components of robotic drive 24.

In one embodiment a torquer 102 for an elongated medical device 220 includes a body having a cavity defining a pathway. In one implementation the body includes proximal housing member 108 and distal housing member 110 each having a cavity therein. Referring to FIG 5 and FIG 11, EMD 220 extends through the pathway defined by an opening or channel through fastener 162, knob 160, biasing member 178, drive gear 172, shaft 150, proximal housing member 108, biasing member 124, pusher 112, distal housing member 110 and guide tube 208. A first pad 120 is movable within the cavity. A biasing member 178 separate from the first pad 120 biases the first pad 120 relative to the body. An actuator is movable relative to the body moving the first pad pinching and/or unpinching the elongated medical device with the first pad within the pathway. A knob is releasably connected to the actuator upon an application of a predetermined torque exceeding a predetermined value.

Although the present disclosure has been described with reference to example embodiments, workers skilled in the art will recognize that changes may be made in form and detail without departing from the spirit and scope of the defined subject matter. For example, although different example embodiments may have been described as including one or more features providing one or more benefits, it is contemplated that the described features may be interchanged with one another or alternatively be combined with one another in the described example embodiments or in other alternative embodiments. The present disclosure described is manifestly intended to be as broad as possible. For example, unless specifically otherwise noted, the definitions reciting a single particular element also encompass a plurality of such particular elements.

## Claims

1. A device for manipulating an elongated medical device comprising:
a torquer releasably securing an elongated medical device (EMD) thereto; and
a torque limiting actuator limiting a torque applied to the torquer.

2. The device of claim 1, wherein the torque limiting actuator limits the torque applied to the torquer to a predetermined torque.

3. The device of claim 1, wherein the torque limiting actuator includes a manual operator and a clutch that prevents an application of torque to the torquer beyond a predetermined torque.

4. The device of claim 1, wherein the torque limiting actuator includes a knob being manually rotated about a longitudinal axis of the torquer, a biasing member between a portion of the knob and a first clutch member, wherein the first clutch member imparts torque to a second clutch member until a predetermined torque between the first clutch member and second clutch member is reached.

5. The device of claim 1, wherein the torque limiting actuator limits the torque applied to the actuator in a first direction but does not limit the torque applied to the actuator in a second opposite direction.

6. The device of claim 1, wherein the torquer includes a torquer body and a pusher movable within the torquer body to move at least a first pad in a direction perpendicular to a longitudinal axis of a torquer to pinch the EMD.

7. The device of claim 1, wherein a pinch force perpendicular to a longitudinal axis of the torquer between the torquer and the EMD is a function of the torque applied by the torque limiting actuator to the torquer.

8. The device of claim 3, wherein the clutch is a spring biased first gear that engages a second gear.

9. The device of claim 8, wherein the spring biased first gear slips relative to the second gear once a predetermined torque between the first gear and the second gear is exceeded.

10. The device of claim 6, wherein the torquer includes a second pad spaced from and movable toward and away from the first pad to releasably pinch the EMD.

11. The device of claim 10, further including a biasing member having a pair of arms spaced from one another and spaced from a longitudinal axis of the torquer.

12. The device of claim 1, wherein the torque limiting actuator includes a shaft having a portion threadedly secured to a body of the torquer and a distal portion operatively moving a pad into engagement with the EMD upon rotation of the shaft relative to the body.

13. The device of claim 12, wherein the torque limiting actuator includes a drive gear and a biasing member biasing the drive gear into engagement with a driven gear secured to the shaft, the drive gear.

14. The device of claim 13, wherein the torque limiting actuator includes a knob manually rotated relative to the body, the knob being releasably connected to the shaft upon an application of a predetermined torque.

15. The device of claim 1, wherein the torque limiting actuator provides an indication to a user once adequate torque has been applied to the torquer.

16. A torquer for an elongated medical device:
a body having a cavity defining a pathway;
a first pad movable within the cavity;
a biasing member separate from the first pad biasing the first pad relative to the body;
an actuator movable relative to the body moving the first pad, wherein movement of the first pad pinches and/or unpinches the elongated medical device, with the first pad, within the pathway; and
a knob releasably connected to the actuator upon an application of a predetermined torque exceeding a predetermined value.

17. An EMD drive system comprising:
a robotic drive having a robotic drive longitudinal axis;
a device module movable along the robotic drive longitudinal axis; and
a drive train coupling a motor to a driven member configured to rotate a torquer pinching an elongated medical device (EMD) about an EMD longitudinal axis, the torquer including a torque limiting actuator being manually accessible to a user when the torquer is in an in-use position in the drive module.

18. The EMD drive system of claim 17, wherein the torque limiting actuator limits the torque applied to the torquer to a predetermined torque.

19. The EMD drive system of claim 17, wherein the torque limiting actuator includes a manual operator and a clutch that prevents an application of torque to the torquer beyond a predetermined torque.

20. The EMD drive system of claim 17, wherein the torque limiting actuator includes a knob being manually rotated about a longitudinal axis of the torquer, a biasing member between a portion of the knob and a first clutch member, wherein the first clutch member imparts torque to a second clutch member until a predetermined torque between the first clutch member and second clutch member is reached.

21. The EMD drive system of claim 17, wherein the torquer may be moved from a first device module to a second device module.

22. The EMD drive system of claim 17, wherein the torquer includes a torque limiting actuator that limits the torque applied to the drive train beyond a predetermined torque.
